# EUROPEAN PATENT APPLICATION

(11) **EP 2 884 328 A1**
(43) Date of publication of application: **17.06.2015**
(21) Application number: 14193539.5
(22) Date of filing: 17.11.2014
(51) Int. Cl.: G02B 27/00, G02B 26/12, G06F 3/01, G06K 9/00, H04N 1/113, H04N 1/193, H04N 3/08, A61B 3/113

(54) **Line scan camera eye tracking system and method**

(30) Priority: 13.12.2013 US 201314106306
(71) Applicant: Honeywell International Inc., Morristown, NJ 07962-2245 (US)
(72) Inventor: Kotaba, Ondrej, Morristown, NJ New Jersey 07962-2245 (US)
(74) Representative: Houghton, Mark Phillip

(57) **Abstract**

A system and method for determining positions of one or more eyes in a three-dimensional volume includes rotating an optical reflector within a linear field of view of a line scan camera to allow the line scan camera to capture image data of a two-dimensional projection within the three-dimensional volume, and processing the image data captured by the line scan camera to determine the positions of the one or more eyes.

## Description

### TECHNICAL FIELD

The present invention generally relates to eye tracking systems, and more particularly relates to an eye tracking system and method that uses a line scan camera.

### BACKGROUND

Presently known eye or gaze tracking systems are typically implemented using one of three general configurations. The first configuration utilizes a head-mounted camera that is pointed directly at the eye. The second configuration uses a set of one or more fixed-mounted overview cameras and a servo-controlled, long-focal-length camera directed at the eye. The third configuration uses a single fixed mounted camera.

In general, the first configuration is significantly less complex than the second, but it does exhibit certain drawbacks. For example, it can be difficult to maintain proper calibration, since even slight motions of the camera with respect to the eye can cause relatively large estimation errors. The head-mounted camera can also be uncomfortable to wear and can obstruct the wearer's field of view. In most instances, image data from the head-mounted camera is transferred via a high-speed bus, resulting in a cable being connected to the wearer's head. While wireless data transfer is technically feasible, the added weight of a battery and requisite recharge can be obtrusive. This configuration also relies on a separate camera for each eye.

The second configuration, which is typically used in a laboratory environment, also exhibits drawbacks. In particular, it, too, can be difficult to maintain proper calibration if disposed within environments with significant vibration, and relies on a separate camera for each eye. The servomechanism may not be able to withstand the vibrations in an aircraft cockpit, and the long-focal-length camera would need to be rigidly mounted to achieve sufficient precision in a vibrating environment, thereby increasing the servomechanism size and power.

The third configuration, in the current state of the art, has a significantly limited accuracy and field of view, both due to very limited angular resolution of the acquired image.

Hence, there is a need for an eye tracking system that can maintain its calibration in a vibrating environment, such as an aircraft cockpit, need not be worn by a user, and/or does not rely on a separate camera for each eye. The present invention addresses at least these needs.

### BRIEF SUMMARY

This summary is provided to describe select concepts in a simplified form that are further described in the Detailed Description. This summary is not intended to identify key or essential features of the claimed subject matter, nor is it intended to be used as an aid in determining the scope of the claimed subject matter.

In one embodiment, an eye tracking system includes a line scan camera, an optical reflector, and a processor. The line scan camera has a linear field of view and is configured to capture image data within the linear field of view. The optical reflector is disposed within the linear field of view. The optical reflector is adapted to be rotated and is configured, when rotating, to allow the line scan camera to capture image data of a two-dimensional projection within a three-dimensional volume. The processor is coupled to receive the image data captured by the line scan camera and is configured, upon receipt thereof, to determine positions of one or more eyes within the three-dimensional volume.

In another embodiment, a method for determining positions of one or more eyes in a three-dimensional volume includes rotating an optical reflector within a linear field of view of a line scan camera to allow the line scan camera to capture image data of a two-dimensional projection within the three-dimensional volume, and processing the image data captured by the line scan camera to determine the positions of the one or more eyes.

In yet another embodiment, an eye tracking system for a vehicle interior includes a line scan camera, an optical reflector, and a processor. The line scan camera has a linear field of view and is configured to capture image data within the linear field of view. The optical reflector is disposed within the linear field of view. The optical reflector is adapted to be rotated and is configured, when rotating, to allow the line scan camera to capture image data within the vehicle. The processor is coupled to receive the image data captured by the line scan camera and is configured, upon receipt thereof, to determine positions of one or more eyes within the aircraft cockpit and a direction of gaze of the one or more eyes.

Furthermore, other desirable features and characteristics of the eye tracking system and method will become apparent from the subsequent detailed description and the appended claims, taken in conjunction with the accompanying drawings and the preceding background.

### BRIEF DESCRIPTION OF THE DRAWINGS

The present invention will hereinafter be described in conjunction with the following drawing figure, wherein like numerals denote like elements, and wherein:
FIG. 1 depicts a functional block diagram of one example embodiment of a line scan camera eye tracking system; and
FIG. 2 depicts a simplified representation of how the system of FIG. 1 detects both eye position and gaze direction.

### DETAILED DESCRIPTION

The following detailed description is merely exemplary in nature and is not intended to limit the invention or the application and uses of the invention. As used herein, the word "exemplary" means "serving as an example, instance, or illustration." Thus, any embodiment described herein as "exemplary" is not necessarily to be construed as preferred or advantageous over other embodiments. All of the embodiments described herein are exemplary embodiments provided to enable persons skilled in the art to make or use the invention and not to limit the scope of the invention which is defined by the claims. Furthermore, there is no intention to be bound by any expressed or implied theory presented in the preceding technical field, background, brief summary, or the following detailed description.

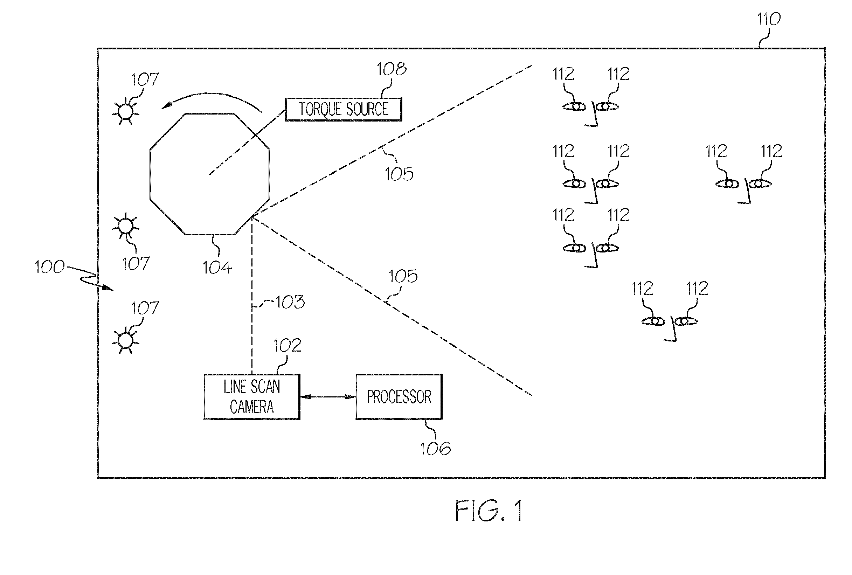

Referring to FIG. 1, a functional block diagram of one embodiment of an eye tracking system 100 is depicted and includes a line scan camera 102, an optical reflector 104, and a processor 106. The line scan camera 102, as is generally known, has a single row of pixel sensors, instead of a matrix of sensors, and thus has a linear field of view (FOV) 103. The line scan camera 102 is configured to capture image data within its linear field of view and supply this image data to the processor 106. It will be appreciated that the line scan camera 102 may be implemented using any one of numerous line scan cameras now known or developed in the future, and may have varying levels of resolution, as needed or desired..

The optical reflector 104 is disposed within the linear field of view of the line scan camera 102. The optical reflector 104 is coupled to receive a drive torque from a drive torque source 108. The optical reflector 104 rotates in response to the drive torque. As FIG. 1 clearly depicts, the optical reflector 104, when rotating, effectively increases the FOV 105 of the line scan camera 102, allowing it to capture image data of a two-dimensional projection of a three-dimensional volume 110, such as an aircraft cockpit. The optical reflector 104 may be variously implemented and configured. For example, the optical reflector may be implemented as a prism or a mirror, just to name a few. In some embodiments, the optical reflector 104 could be a non-uniform device, whereby various frames sensed by the line scan camera 102 could have different properties (e.g., resolution, FOV, etc.). As FIG. 1 also depicts, one or more illumination sources 107, which may emit light the in narrow infrared band, may be used to illuminate the three-dimensional volume 110 and serve as an angular reference to determine direction of gaze.

The processor 106 is coupled to receive the image data captured by the line scan camera 102 and is configured, upon receipt thereof, to determine the positions of one or more eyes 112 within the three-dimensional volume 110. The specific manner in which the processor 106 determines the positions of the one or more eyes may vary, but in a particular preferred embodiment it does so by decimating the image data to achieve a first, relatively low, level of image resolution. his is possible since high resolution image processing is not needed to determine the positions of the one or more eyes 112. In some embodiments, the processor 106 is additionally configured to determine the direction of gaze of the one or more eyes 112. To implement this functionality, the processor 106 is further configured to extract the image data around the positions of the one or more eyes 112 and process these data to achieve a second, relatively high, level of image resolution. As may be appreciated, the second level of image resolution is greater than the first level of image resolution.

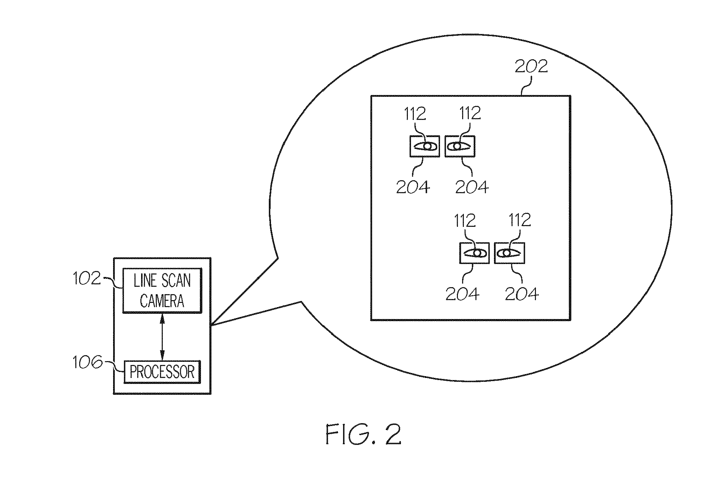

The above-described processes are illustrated, in simplified form, in FIG. 2. A simplified representation of an image 202 captured by the line scan camera 102 in the increased FOV 105 is depicted. The processor 106, as noted above, decimates the image data associated with this image 202 to determine the positions of the eyes 112. As FIG. 2 also depicts, the regions 204 around the eyes 112 are processed at a higher image resolution, so that the direction in which each eye 112 is gazing may be determined.

The system and method described herein provides significant advantages over presently known eye tracking systems. The system 100 is of relatively non-complex, has a relatively low weight, and is relatively robust and insensitive to vibrations. The line scan camera 102 and processor 106 allow both relatively high-resolution images of the eyes 112 and relatively low-resolution images of the remainder of the three-dimensional volume 110 to be achieved from a single set of image data. As such, the system 100 is relatively insensitive to alignment errors, vibrations, and timing errors. The system 100 may track numerous numbers of eyes 112, and is limited only by the processing speed. A desired combination of resolution, fps (frames-per-second) and field-of-view (FOV) may be readily achieved by selecting an appropriate optical reflector and shape. Moreover, the rotation speed of the optical reflector 104 may be adjusted to modify the resolution in one axis and the fps in runtime. Moreover, because relatively low resolution processing may be used to determine the positions of the one or more eyes 112, and high resolution processing is only used around the determined eye positions to determine gaze directions, significant processing power can be saved.

Those of skill in the art will appreciate that the various illustrative logical blocks, modules, circuits, and algorithm steps described in connection with the embodiments disclosed herein may be implemented as electronic hardware, computer software, or combinations of both. Some of the embodiments and implementations are described above in terms of functional and/or logical block components (or modules) and various processing steps. However, it should be appreciated that such block components (or modules) may be realized by any number of hardware, software, and/or firmware components configured to perform the specified functions. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention. For example, an embodiment of a system or a component may employ various integrated circuit components, e.g., memory elements, digital signal processing elements, logic elements, look-up tables, or the like, which may carry out a variety of functions under the control of one or more microprocessors or other control devices. In addition, those skilled in the art will appreciate that embodiments described herein are merely exemplary implementations.

The various illustrative logical blocks, modules, and circuits described in connection with the embodiments disclosed herein may be implemented or performed with a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general-purpose processor may be a microprocessor, but in the alternative, the processor may be any conventional processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration.

The steps of a method or algorithm described in connection with the embodiments disclosed herein may be embodied directly in hardware, in a software module executed by a processor, or in a combination of the two. A software module may reside in RAM memory, flash memory, ROM memory, EPROM memory, EEPROM memory, registers, hard disk, a removable disk, a CD-ROM, or any other form of storage medium known in the art. An exemplary storage medium is coupled to the processor such that the processor can read information from, and write information to, the storage medium. In the alternative, the storage medium may be integral to the processor. The processor and the storage medium may reside in an ASIC. The ASIC may reside in a user terminal. In the alternative, the processor and the storage medium may reside as discrete components in a user terminal.

In this document, relational terms such as first and second, and the like may be used solely to distinguish one entity or action from another entity or action without necessarily requiring or implying any actual such relationship or order between such entities or actions. Numerical ordinals such as "first," "second," "third," etc. simply denote different singles of a plurality and do not imply any order or sequence unless specifically defined by the claim language. The sequence of the text in any of the claims does not imply that process steps must be performed in a temporal or logical order according to such sequence unless it is specifically defined by the language of the claim. The process steps may be interchanged in any order without departing from the scope of the invention as long as such an interchange does not contradict the claim language and is not logically nonsensical.

Furthermore, depending on the context, words such as "connect" or "coupled to" used in describing a relationship between different elements do not imply that a direct physical connection must be made between these elements. For example, two elements may be connected to each other physically, electronically, logically, or in any other manner, through one or more additional elements.

While at least one exemplary embodiment has been presented in the foregoing detailed description of the invention, it should be appreciated that a vast number of variations exist. It should also be appreciated that the exemplary embodiment or exemplary embodiments are only examples, and are not intended to limit the scope, applicability, or configuration of the invention in any way. Rather, the foregoing detailed description will provide those skilled in the art with a convenient road map for implementing an exemplary embodiment of the invention. It being understood that various changes may be made in the function and arrangement of elements described in an exemplary embodiment without departing from the scope of the invention as set forth in the appended claims.

## Claims

1. An eye tracking system, comprising:
a line scan camera having a linear field of view and configured to capture image data within the linear field of view;
an optical reflector disposed within the linear field of view, the optical reflector adapted to be rotated and configured, when rotating, to allow the line scan camera to capture image data of a two-dimensional projection within a three-dimensional volume; and
a processor coupled to receive the image data captured by the line scan camera and configured, upon receipt thereof, to determine positions of one or more eyes within the three-dimensional volume.

2. The system of claim 1, further comprising:
one or more illumination sources to configured to emit light to illuminate the three-dimensional volume.

3. The system of claim 3, wherein the one or more light sources also serve as an angular reference for gaze detection.

4. The system of claim 1, wherein the processor is configured to:
determine the positions of the one or more eyes by decimating the image data to achieve a first level of image resolution; and

5. The system of claim 1, wherein the processor is further configured to determine a direction of gaze of the one or more eyes.

6. The system of claim 5, wherein the processor is configured to:
determine the positions of the one or more eyes by decimating the image data to achieve a first level of image resolution; and
determine the direction of gaze by extracting image data around the positions of the one or more eyes to achieve a second level of image resolution, the second level of image resolution being greater than the first level of image resolution.

7. The system of claim 1, wherein the optical reflector is a non-uniform optical reflector.

8. The system of claim 1, wherein the optical reflector comprises a mirror.

9. The system of claim 1, wherein the optical reflector comprises a prism.

10. A method for determining positions of one or more eyes in a three-dimensional volume, comprising the steps of:
rotating an optical reflector within a linear field of view of a line scan camera to allow the line scan camera to capture image data of a two-dimensional projection within the three-dimensional volume; and
processing the image data captured by the line scan camera to determine the positions of the one or more eyes.
